# EUROPEAN PATENT APPLICATION

(11) **EP 0 524 910 A2**
(43) Date of publication of application: **27.01.1993**
(21) Application number: 92810566.7
(22) Date of filing: 23.07.1992
(51) Int. Cl.: C12N 15/55, C12N 15/82, C12N 15/11, A01H 5/00

(54) **Modulation of flower colour**

(30) Priority: 25.07.1991 GB 9116071
(71) Applicant: SANDOZ LTD., CH-4002 Basel (CH); SANDOZ-PATENT-GMBH, D-79539 Lörrach (DE)
(72) Inventor: van Holst, Gerrit-Jan, NL-1602 DN Enkhuizen (NL); Kootstra, Jille Jan, NL-1602 GC Enkhuizen (NL); van Grinsven, Martinus Quirinus Joseph Marie, NL-1602 MA Enkhuizen (NL); Schram, Andre Willem, NL-1402 AH Bussum (NL)

(57) **Abstract**

DNA constructs capable of influencing pigment colour in the vacuoles of flower cells comprising DNA sequences coding for transcription into RNA sequences encoding vacuolar ATPase subunits, DNA sequences coding for transcription into RNA sequences homologous to RNA sequences encoding vacuolar ATPase subunits, and DNA sequences coding for RNA sequences complementary to such RNA sequences wherein the DNA constructs are under the control of promoter and terminators capable of functioning in plants.

## Description

The present invention relates to plants having an altered vacuolar pH resulting in a modification of the colour of flower pigments which display a spectral dependency on vacuolar pH, genetic material employed in generating this modification, and processes for obtaining such plants and genetic material.

### Background

A major aim in flower breeding is to provide the market with a broad palette of flower colours. Breeders use naturally pigmented varieties within species and combine plant genomes with a desired genetic (background) history by employing conventional breeding techniques. However, modern biotechnology strategies have been proposed to increase the diversity of flower colouring in plants.

The present invention provides for methods of modifying the expression of flower anthocyanins or anthocyanidins by modulation of the vacuolar pH in flowers.

Internal vacuolar pH is generally low, having values in the range of pH 3-5. The absorption spectrum of anthocyanins is highly sensitive to pH changes. For example an increase of 0.5 of the vacuolar pH leads to a significant change in flower colour from for example red to blue. In Petunia hybrida several genes have been identified which do influence vacuolar pH and thus the colour of the flower.

In Petunia hybrida for example, genetic loci have been identified which influence vacuolar pH and thus the colour of the flower [Monographs on Theoretical and Applied Genetics 9, Petunia. Ed. Sink K. C., Springer-Verlag, Berlin, pp 49-67 (1984)].

Certain vacuolar ATPase subunit genes are known and have been described for carrot [Zimniak L. et al (1988) J. Biol. Chem. 5 : 9102-9112].

Without intending to be bound by theory, it is thought that plant internal vacuolar pH may be maintained by perhaps one or more factors such as tonoplast ATPase, tonoplast pyrophosphatase, and antiporters which exchange positive metal ions for protons across the vacuolar membrane. Moreover, buffering capacity of vacuolar sap also contributes in the maintenance of intravacuolar pH.

Tonoplast ATPase is thought to play an important role. This multi-subunit protein consists of membrane-spanning subunits and polypeptide chains exposed to the exterior of the vacuole. Tonoplast ATPase genes encode for large multimeric enzymes of about 500 kd composed of an integral membrane moiety and a hydrophilic catalytic complex. The hydrophilic complex contains up to five different subunits (Nathan N. and Taiz L. TIBS 14 March 1989 pp 113-116). On hydrolysing cytosolic ATP, protons are pumped into the vacuole where they serve to increase the internal acidity of the vacuole.

An object of the invention comprises influencing the colour of flower pigments localised in the plant vacuoles, which pigments display a spectral dependency on vacuolar pH. In this respect, it is thought that the primary chemical structure and function thereof of the plant pigment remains essentially unchanged, however, the physical characteristic of light absorption, alters with increasing pH (and correspondingly with decreasing pH). As the pH level increases from acidic through to basic, there is a corresponding shift in absorption giving rise to altered colouring from red to blue (should the pH value be decreased from basic through to acidic there would be an apparent corresponding shift in absorption giving rise to altered colouring from blue to red). Thus, shades of desirable colours discernible within the visible light spectrum may be attained in the flowers. Shifts from red to blue may be accomplished by inhibiting the activity of vacuolar ATPases and thus inhibit acidification within the vacuole. This in turn can lead to an increase in vacuolar pH and changes in pigment colouring and therefore flower colour.

Another object of the invention is to provide a method of altering vacuolar pH, such as by increasing vacuolar pH in flowers, by employing recombinant DNA molecules which comprise DNA sequences coding for a vacuolar ATPase subunit gene in a sense or antisense direction and modifications thereof. Such a method may involve inhibiting the vacuolar ATPase or reducing or inhibiting the synthesis thereof.

Reduction or inhibition of the synthesis of vacuolar ATPase may be achieved by utilising a DNA construct which comprises a vacuolar ATPase subunit nucleotide sequence or gene constructed in a sense or anti-sense direction. Over-expression of a sense ATPase subunit DNA sequence or gene may lead to an increased synthesis of a subunit component which contributes to the vacuolar ATPase and thereby cause an improper assembly of vacuolar ATPase. The introduction of an anti-sense construct into a plant genome can lead to a decrease in the synthesis of vacuolar ATPase.

Inactivation of vacuolar ATPase may also be achieved through the use of a DNA construct comprising a mutated ATPase subunit which can result in improper folding within the ATPase multimeric enzyme complex and loss of ATP hydrolytic activity.

### Detailed Description

According to the present invention there are provided recombinant DNA constructs capable of influencing pigment colour in the vacuoles of flower cells which comprise a DNA sequence coding for transcription
a) into an RNA sequence which encodes a vacuolar ATPase subunit or a part thereof; or into an RNA sequence homologous with an RNA sequence which encodes a vacuolar ATPase subunit or encodes a part of a vacuolar ATPase subunit; or
b) into an RNA sequence complementary to an RNA sequence which encodes a vacuolar ATPase subunit or a part thereof; or into an RNA sequence homologous with an RNA sequence complementary to an RNA sequence which encodes a vacuolar ATPase subunit or encodes a part of a vacuolar ATPase subunit;

which DNA sequence is under the expression control of a promoter capable of functioning in plants and includes a terminators capable of functioning in plants.

The "DNA constructs capable of influencing pigment colour in the vacuoles of flower cells" according to the invention are constructs which when present in the plant genome interfere with the normal operation, activity, assembly or expression of vacuolar ATPase such that the vacuolar pH value is altered and results in changes in vacuolar pigments sensitive to said pH alterations, and thus changes in the colour of flowers. Preferably, vacuolar pH is elevated, i.e. becomes more basic, by the inclusion of DNA constructs of the invention into the plant genome which DNA constructs have an effect on ATPase expression such that flower colour can be altered from red hues to blue hues.

The DNA sequences defined under a) and b) above, hereinafter are referred to as "ATPase related DNA sequences". ATPase related DNA sequences include inter alia mutants or other modifications of parts or a part of a DNA sequence encoding a vacuolar ATPase protein, an ATPase subunit protein or sequences homologous thereto. Such modified proteins may be obtained by conventional means, for example, by site-directed mutagenesis.

The "term RNA sequence homologous to an RNA sequence which encodes a vacuolar ATPase subunit" refers to an RNA sequence of a vacuolar ATPase subunit gene wherein a number of nucleotides have been substituted, deleted and/or added but which is capable of hybridisation to a nucleotide sequence complementary to an RNA sequence of that vacuolar ATPase subunit gene. In this context, hybridisation refers to hybridisation under appropriate, i.e. conventional hybridisation conditions. For the purpose of the present invention, appropriate hybridisation conditions include preferably an incubation period of about 16 hours at 42°C, in a buffer system comprising 5 x standard saline citrate (SSC), 0.5 % sodium dodecylsulphate (SDS), 5 x Denhardt's solution, 50 % formamide and 100 µg/ml carrier DNA (hereinafter the buffer system), followed by washing 3 times with a buffer comprising 1 x SSC and 0.1 % SDS at 65°C for about one hour per wash.

More preferred hybridisation conditions for the purpose of the present invention involve incubation in the buffer system for 16 hours at 49°C and washing 3 times with a buffer comprising 0.1 x SSC and 0.1 % SDS at 55°C for about one hour per wash. Most preferred hybridisation conditions for the purpose of the invention involve incubation in the buffer system for 16 hours at 55°C and washing 3 times with a buffer comprising 0.1 x SSC and 0.1 % SDS at 65° for about one hour per wash.

The DNA sequences coding for transcription into a part of an RNA sequence encoding a vacuolar ATPase subunit defined under a) above, will conveniently code for 30 % or more of the length (in terms of number of amino acids) of an ATPase subunit protein.

The DNA sequences coding for transcription into an RNA sequence homologous with an RNA sequence which encodes a vacuolar ATPase subunit or a part of a vacuolar ATPase subunit as defined under a) above conveniently code for a protein having substantial sequence homology to a vacuolar ATPase subunit or to a part of a vacuolar ATPase subunit, typically at least 70 %, preferably at least 80 %, more preferably at least 90 % homology. Any DNA sequences coding for transcription into an RNA sequence homologous with an RNA sequence encoding a part of a vacuolar ATPase subunit, will conveniently code for a protein having at least 30 % or more of the length of an ATPase subunit protein.

Preferably the DNA sequence defined under a) above code for a protein having a length of from between 80 % to 120 % of the length of an ATPase subunit protein. Most preferably said DNA sequences code for a protein having a length of from between 90 % to 110 % of the length of an ATPase subunit protein.

The length of the ATPase related DNA sequences according to b) above typically lies within the range of from 50 nucleotides up to 120 %, more preferably from 200 nucleotides up to 110 %, more preferably from 200 nucleotides to 100 % of the number of nucleotides included in the DNA sequence encoding a vacuolar ATPase subunit.

The term "promoter" as used herein refers to the nucleotide sequence upstream from the translational start site and containing all the regulatory regions required for transcription including the region coding for the leader sequence of mRNA (which leader sequence comprises the ribosomal binding site and initiates translation at the AUG start codon).

Examples of promoters suitable for use in DNA constructs of the invention include viral, fungal, bacterial, animal and plant derived promoters capable of functioning in plant cells. The promoter may express the DNA constitutively or more preferably differentially. Suitable examples of promoters which are capable of differentially regulating DNA expression are promoters expressed in specific cell types such as flower specific promoters, e.g. the chalcone synthase (CHS) promoter, the chalcone isomerase (CHI) promoter, the dihydroflavonol reductase (DFR) promoter or other so-called tissue specific promoters. It will be appreciated that the promoter employed should give rise to the expression of an ATPase Related DNA Sequence at a Rate sufficient to produce the amount of RNA necessary to alter the vacuolar ATPase activity in the petals of the transformed plant. The necessary amount of RNA to be transcribed may vary with the type of plant involved. Particularly preferred promoters include, but are not limited to, the cauliflower mosaic virus 35S (CaMV 35S) promoter and derivatives thereof, or more preferably promoters active only in flower or petal tissue such as the CHS, CHI or DFR promoter referred to hereinabove.

The term "terminator" as used herein refers to a DNA sequence at the end of a transcriptional unit that signals termination of transcription. Terminators are 3′-non-translated DNA sequences that contain a polyadenylation signal, which cause the addition of polyadenylate sequences to the 3′-end of the primary transcripts. Terminators active in plant cells are known and described in the literature. They may be isolated from for example bacteria, fungi, viruses, animals and plants. Examples of terminators particularly suitable for use in the DNA constructs of the invention include, but are not limited to, the nopaline synthase terminator of A. tumefaciens, the 35S terminator of CaMV and the zein terminator from Zea mays.

For the purpose of the present invention, an RNA sequence is complementary to another RNA sequence if it is able to form a hydrogen-bonded complex with it, according to rules of base pairing under appropriate hybridisation conditions as defined hereinabove.

The present invention also provides a vector capable of introducing DNA constructs of the invention into plants and methods of producing such vectors.

The term "vector" employed herein refers to a vehicle with which DNA fragments can be incorporated into the genome of a host organism and which containing a promoter and a terminator capable of functioning in plant cells.

The term "plants" herein is employed in a wide sense and refers to fully differentiated plants as well as undifferentiated plant material such as protoplasts, plant cells, seeds, plantlets and the like which under appropriate conditions can develop into mature plants, the progeny thereof and parts thereof such as cuttings and fruits of such plants.

The invention further provides plants comprising in their genome a DNA construct of the invention, and methods of producing such plants.

The plants according to the invention have an altered vacuolar pH due to the insertion into their genome of an ATPase Related DNA Sequence according to the invention and do not have the disadvantages and limitations of plants obtained by classical methods as discussed hereinabove.

**Examples of** plants which may be genetically modified, include but are not limited to ornamentals such as Anthirrhinum, Chrysanthemum, Cyclamen, Dahlia, Datura, Delphinium, Gerbera, Gladiolus, Gloxinia, Hippeastrum, Impatiens, Mesembryanthemum, Papaver, Pelargonium, Primula, Rosa, Saint Paulia, Salpiglossis, Helianthus (sunflower), Tagetes, Verbena and Vinca.

Preferred DNA constructs of the invention comprise:
(i) the DNA sequence (SEQ ID No: 1) encoding the 69 kd subunit of vacuolar ATPase obtainable from carrot (SEQ ID No. 2) or a part thereof; or an equivalent DNA sequence coding for said ATPase part thereof; **or**
(ii) a DNA sequence coding for transcription into an RNA sequence complementary to the RNA sequence encoding the 69 kd subunit of vacuolar ATPase obtainable from carrot, or a part thereof; or a DNA sequence coding for transcription into an RNA sequence homologous with the RNA sequence complementary to the RNA sequence encoding the 69 kd subunit of vacuolar ATPase obtainable from carrot, or a port thereof.

The DNA constructs of the invention may be obtained by insertion of the ATPase Related DNA sequence in an appropriate vector, such that it is brought under expression control of a promoter capable of functioning in plants and its transcription terminated by a terminator capable of functioning in plants.

The insertion of the ATPase Related DNA Sequence in an appropriate vector may be carried out in a manner known per se. Suitable procedures are also illustrated in the examples hereinafter.

Likewise the construction of an appropriate vector may be carried out in a manner known per se.

The plants according to the invention may be obtained by
a) inserting into the genome of a plant cell a DNA construct according to the invention;
b) obtaining transformed cells; and
c) regenerating from the transformed cells genetically transformed plants.

The DNA vectors of the invention may be inserted into plant genomes. Such plant transformations may be carried out employing techniques known per se for the transformation of plants, such as the plant transformation techniques involving the Ti plasmids derived from Agrobacterium tumefaciens, A. rhizogenes or modifications thereof, naked DNA transformation or electroporation of isolated plant cells or organised plant structures, the use of micro projectiles to deliver DNA, the use of laser systems, liposomes, or viruses or pollen as transformation vectors and the like.

The plants of the invention may be monitored for expression of an ATPase Related DNA sequence by methods known in the art such as Northern analysis, Southern analysis, PCR techniques and/or immunological techniques and the like. The plants of the invention show altered vacuolar pH as demonstrable through procedures known to the skilled artisan.

Methods suitable for determining the pH of plant vacuoles are known in the art. Suitable procedures are described in the examples hereinafter.

The plants of the invention may also be obtained by crossing a plant obtained according to the methods of the invention with another plant to produce plants having in their plant genome a DNA construct of the invention.

The invention is illustrated by the following non-limiting examples and attached figures.

**Figure** **1:** is a schematic presentation of the preparation of pZU 129 according to Example 2.

**Figure 2:** is a schematic presentation of the preparation of vector pZU-C according to Example 4.2.

**Figure 3:** is a schematic presentation of the preparation of vectors pZU 145 A and pZU 145 B according to Example 5.3.

**Figure 4:** is a schematic presentation of the preparation of vectors pZU 150 A and pZU 150 B according to Example 5.3.

Other objects, features and advantages of the present invention will become apparent from the following examples.

### Material and Methods

All RNA derived sequences herein are depicted as DNA sequences for the sole purpose of uniformity. A person skilled in the art appreciates that this is done for convenience only.

Cultivars of Petunia hybrida, used in plant transformation studies, are grown under standard greenhouse conditions. Axenic explant material is grown on standard MS media [Murashige and Skoog, (1962) Physiol. Plant 15: 473-497] containing appropriate phytohormones and sucrose concentrations.

E. coli bacteria are grown on rotary shakers at 37°C in standard LB-medium. Agrobacterium tumefaciens strains are grown at 28°C in Min A medium supplemented with 0.1 % glucose [Ausubel et al., (1987) Current protocols in Molecular Biology (Green Publishing Associates and Wiley Intersciences, New York].

In all cloning procedures, the E. coli strain JM83, (F-, Δ (lac-pro), ara, rpsL, ⌀80, dlacZM15) is used as a recipient for recombinant plasmids.

Binary vectors are conjugated to Agrobacterium tumefaciens strain LBA 4404, a strain containing the Ti-plasmid vir region, [Hoekema et al., (1983) Nature 303: 179-180] in standard triparental matings using E. coli HB101, containing the plasmid pRK2013 as a helper strain. [Figurski and Helinski (1979), Proc. Natl. Acad. Sci. USA 76: 1648-1652] Appropriate Agrobacterium tumefaciens recipients are selected on media containing rifampicin (50 µg/ml) and kanamycine (50 µg/ml).

Cloning of fragments in the vectors pUC19 [Yanish-Perron et al., (1985), Gene 33: 103-119], pBluescript (Stratagene), pBIN19 [Bevan et al., (1984), Nucl. Acids. Res. 12: 8711-8721] or derivatives, restriction enzyme analysis of DNA, transformation to E. coli recipient strains, isolation of plasmid DNA on small scale as well as large scale, nick-translation, in vitro transcription, DNA sequencing, Southern blotting, DNA gel electrophoresis, PCR analysis and reversed PCR are performed according to standard procedures [Maniatis et al., (1982) Molecular Cloning, a Laboratory Manual. Cold Spring Harbor Laboratory, N.Y.; Ausubel et al. supra] well known to persons skilled in the art.

For analysis of vacuolar pH, several techniques are known to persons skilled in the art as such and are limited to the methods described below.

### Chemical probes

The technique uses radiolabeled or fluorescent weak bases. Examples of the former are methylamine, benzylamine and nicotine and an example of the latter is 9-aminoacridine. The probe molecules are undissociated outside the cell and diffuse through the cell membrane. An equilibrium is established between the undissociated molecules outside the cell and the dissociated molecules inside the cell. The amount of the probe accumulating inside the cell is a function of extracellular pH, intracellular pH and pKₐ of the probe.

### Microspectrophotomeric measurements

By this method the pH is colourometrically determined, using indicator dyes such as bromophenol blue, bromophenol red, bromocresol green and chlorophenol red. The content of some, e.g. ca. ten vacuoles is collected by means of a micropipette and the content is diluted with indicator dye. The pH is then measured spectrophotometrically.

### H⁺-selective microelectrodes

By using H⁺-selective liquid membrane microelectrodes a fast recording of the vacuolar pH is established.

### ³¹P NMR-spectrometry

This technique relies on the fact that the chemical shift (i.e the resonance frequency relative to a standard) of ³¹P compounds is pH dependent.

### Cell sap pH measurements

Cell sap is extracted from the petals and the pH of the cell sap is measured with a glass electrode.

### Example 1: Isolation of carrot vacuolar 69 kd ATPase subunit gene

**1.1** The carrot vacuolar 69 kd ATPase subunit gene can be isolated from a carrot cDNA library essentially as described by Zimniak et al. (1988), J. Biol. Chem. 5: 9102-9112.
**1.2** Based on the nucleotide sequence presented in the Zimniak paper specific primers are synthesised covering the methione start codon and stopcodon and these primers are used in a reversed PCR experiment to isolate the ± 1872 nucleotide long carrot vacuolar 69 kd ATPase subunit gene from carrot root polyadenylated mRNA. It is also possible to synthesise the gene using the PCR approach in two or more fragments that are subsequently ligated to yield the desired gene.
**1.3** The complete ATPase subunit sequence is synthesised in fragments using commercially available DNA synthesisers. These fragments are ligated to give the desired gene.

The integrity of ATPase subunit gene obtained according to the procedure of examples 1.1, 1.2 and 1.3 is checked via standard DNA sequencing protocols.

### Example 2: cDNA cloning of heterologous ATPase subunit genetic material

A first series of clones is obtained by priming the RNA of carrot with the reverse transcriptase primer: 5'CATGTTCTAG ATCATCGTGT CTCGTCCTCA AG3' (SEQ ID No: 3)
to make cDNA clones. A second primer: 5'CTGAATCTAG AGAAGATCCC TTCTGT3' (SEQ ID No: 4)
is then added with SEQ ID No. 3 to the cDNA mixture for the PCR reaction.

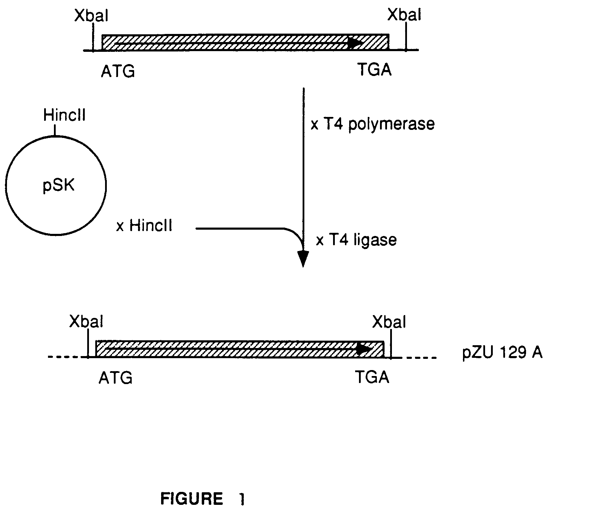

From the resulting PCR mixture, a 1.8 kb PCR fragment is cloned as a Hinc II fragment and then treated with T4 polymerase for blunt end ligation into pBluescript. This results in pZU 129. (Figure 1)

### Example 3: Sequence determination of the heterologous ATPase subunit gene

In order to determine the sequence of the heterologous ATPase subunit genes selected cDNA clones are subcloned in M13mp18. These clones are sequenced in both directions using the standard protocol of Yanish-Perron et al. (1985) supra.

To verify that the cloned gene encodes a heterologous 69 kd ATPase subunit, the open reading frame to be analysed is inserted into pBluescript. Using T7 RNA polymerase the protein gene is transcribed in vitro. This in vitro synthesised transcript is subsequently translated in an in vitro rabbit reticulocyte lysate (New England Nuclear: NEN) system in the presence of [³⁵S] labeled methionine. As a control the carrot 69 kd ATPase subunit gene is inserted into pBluescript, transcribed and translated in vitro and subjected to immunoprecipitation with antibodies raised against purified 69 kd ATPase subunit protein, following procedures essentially as described by Van Grinsven et al (1986) Theor. Appl. Gen 73, 94-101.This indicates that the cloned gene encodes a heterologous 69 kd ATPase subunit gene.

### Example 4: Construction of suitable expression vectors

### Example 4.1 Vector pZU-B

The construction of vector pZU-B is disclosed in EP 0 426 195 A1.

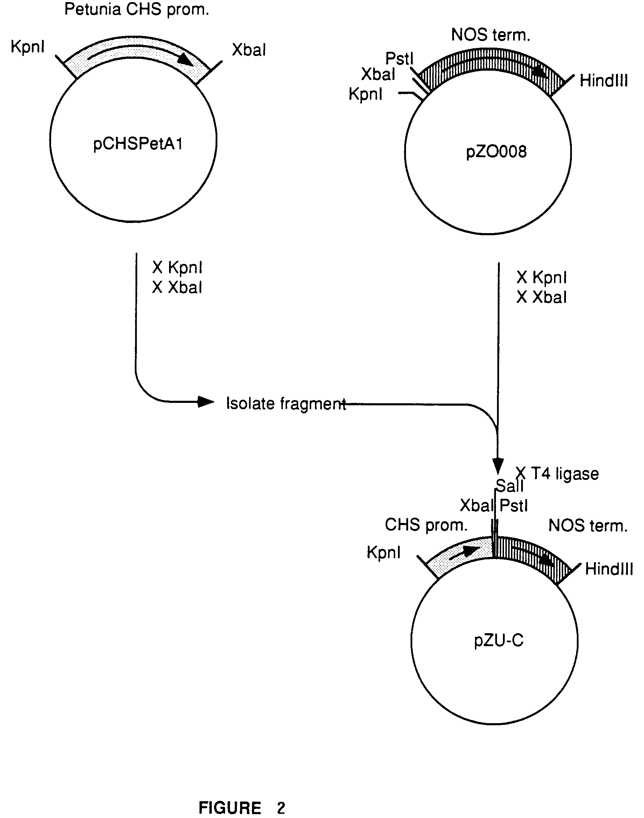

### Example 4.2 Vector pZU-C (Figure 2)

The recombinant plasmid pCHSPetA1 is a derivative of pBR322 carrying Kpn1/Xba1 fragment containing the Petunia hybrida chalcone synthase (CHS) promoter fragment. The CHS promoter fragment is linked to a Xba1 fragment containing the coding region of the maize DFR gene. The terminator sequences present in this vector are derived from the CaMV 35S terminator sequences.

The Kpn1/Xba1 fragment is isolated from pCHSPetA1 and ligated into pZ0008 thus providing pZU-C. The plasmid pZ0008 is disclosed in EP 426 195 and carries the nopaline synthase (NOS) terminator as a 260 pb Pst1-Hind111 fragment.

The resulting recombinant plasmid pZU-C is another plant expression vector. The sequence of CHS promoter as used in the plant expression vector pZU-C is as follows:

1 ATCGATGAGC TTTAAGTGCA TGTGCACTGT CAAATTATGT CATAAAATTC
51 TAATGAGATG AGTTATATAG CATTTACAAA TTCAAATACT TAAAAAGGAT
101 ACTACAATTT GGTATTACCT TGTCAAATTC CTGTTCAAAG CTGATGCTAG
151 GAGTGACAGA AATCATATGT AAGAATGATC AAGACCATTC ATTTGGTTCA
201 ACAGACTGAT GAAAAATCTA AGAGCCTTTG ATCTTGAAGA AGTAGGTAGC
251 GCTAATTCGA AAATAACCAA CCATTAATTT GTTGTGAAGG TTTGCTACGA
301 AAATAAAAAA GGATGTCACG TGCCATCAAG TTATAGCTAC ACGTGATTAC
351 TANCTACCAT TCTTCTTTTG GGCTCTCGTA TAAATACTTT ACAATCCCCA
401 TGCAAACCAT AACACAATAT AAAAGATACT TACACTTGTC ACGTCGCGGA
451 TCC

(SEQ ID No.: 5)

The resulting recombinant plasmid pZU-C contains the CHS promoter, unique SalI, XbqI and PstI sites and the NOS terminator. This expression vector is preferentially used in order to obtain flower specific expression of the ATPase related RNA Sequences.

### Example 5: Construction of plant transformation vectors containing ATPase related sequences

### Example 5.1: Carrot 69 kd ATPase subunit gene constructions in pZU-B

In order to make a fusion in which the ATG start codon from the carrot 69 kd ATPase subunit gene is fused directly to the 3′-end of the TMV untranslated leader of the 35S-Ω promoter the start codon of the carrot 69 kd ATPase subunit gene is mutated. Using site-directed mutagenesis, the sequence 5′ d(CTGAGAAGATGCCT) (SEQ ID No: 6) in the 69 kd ATPase subunit of carrot is mutated to 5′ d(CTGATATCATGCCT) (SEQ ID No: 7), thereby creating an EcoRV site: 5′ d(GAT|ATC) just proximal to the ATG start codon of the carrot 69 kd ATPase subunit gene.

In order to make a fusion in which the TGA stop codon from the carrot 69 kd ATPase subunit gene is fused directly to the 5′-end of the NOS polyadenylation sequences (NOS terminator) the stopcodon of the carrot 69 kd ATPase subunit gene has to be mutated. Using site-directed mutagenesis, the sequence 5′ d(ACACGATGATTGAACATG) (SEQ ID No: 8) is mutated to 5′d(ACACGATGACTGCAGATG) (SEQ ID No: 9), thereby creating an PstI site: 5′d(CTGCA|G) just distal to the TGA stopcodon of the carrot 69 kd ATPase subunit gene. The resulting recombinant plasmid is called pATPmut. The mutated carrot 69 kd ATPase subunit gene is excised from this plasmid via EcoRV-PsI digestion. This fragment is isolated and inserted into the SmaI -PStI linearized pZU-B, resulting in recombinant plasmid, pATPmutB. The chimeric cassette containing the 35S-Ω promoter, the mutated carrot 69 kd ATPase subunit gene and the NOS terminator is excised from pATPmutB via XbaI digestion. The isolated chimeric gene cassette is then inserted into the XbaI linearized pBIN19 to create the binary transformation vector pATPmutBB. The resulting pATPmutBB is used in plant transformation experiments utilizing methods well known to persons skilled in the art.

### Example 5.2: Heterologous 69 kd ATPase subunit gene constructions in pZU-B

The 69 kd ATPase subunit gene is mutated as described and cloned into expression pZU-B as described in Example 5.1. The cassette containing the 35S promoter, the heterologous 69 kd ATPase subunit gene and the NOS terminator is excised from the plasmid by restriction with XbaI and ligated into XbaI linearized pBIN19. The resulting plasmid is used in plant transformation experiments using methods well known to persons skilled in the art.

### Example 5.3: Carrot 69 kd ATPase subunit and complementary 69 kd ATPase subunit gene constructions in pZU-C

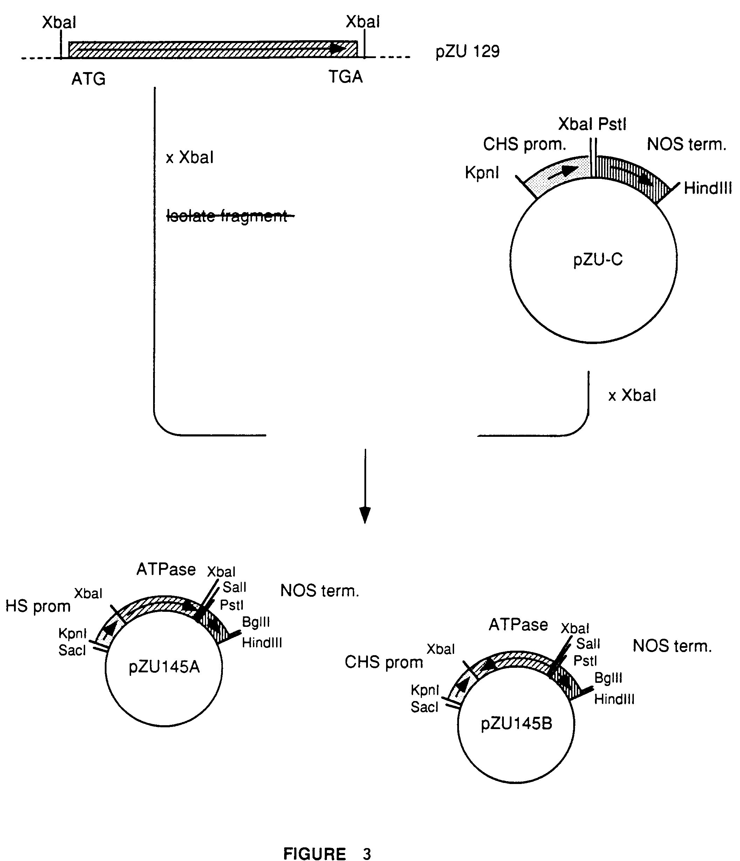

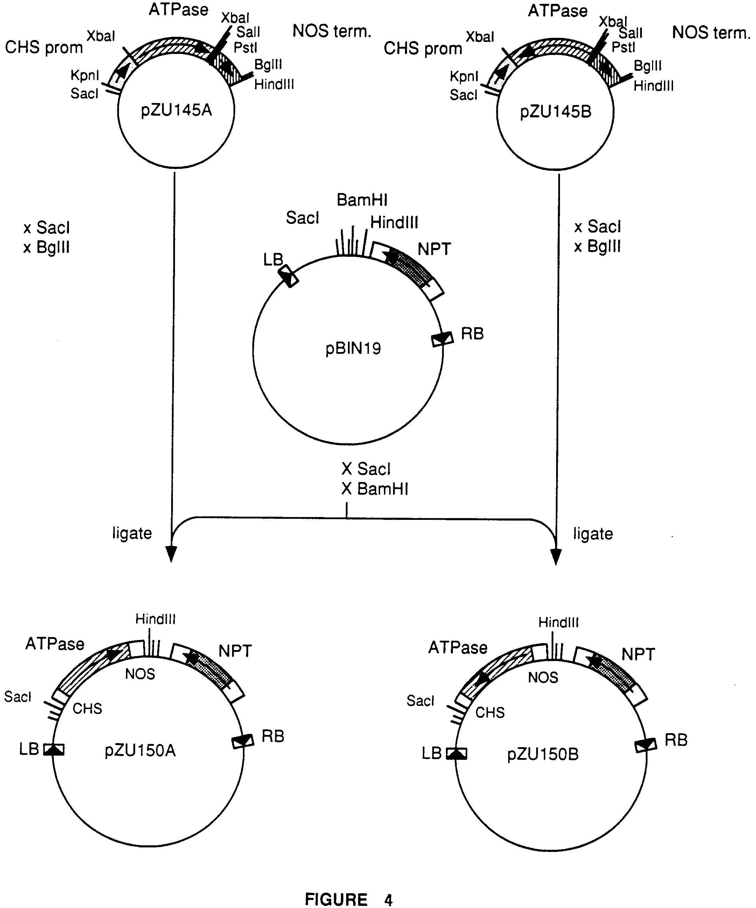

The pZU129 corresponding to carrot 69 kd ATPase subunit is cloned into Xba1 linearized pZU-C. This results in plasmids pZU145A and pZU14)5B (Figure 3). The chimeric CHS-ATPase-NOS cassettes are excised from these plasmids and cloned into appropriate restriction sites of pBIN19, resulting in pZU150A and pZU150B (Figure 4). These plasmids are used in plant transformation experiments utilising methods well known to persons skilled in the art.

### Example 5.4: 69 kd ATPase subunit gene containing a mutation destroying the active site constructions in pZU-C

The active site of the carrot 69 kd ATPase subunit is thought to be located at the Lysine residue 258. Therefore, using site directed mutagenesis this residue is changed into valine, Leucine or Glycine. This mutated ATPase subunit is then cloned into pZU-C and subsequently in pBIN19 to yield the plant transformation vector, essentially as described in Example 5.1. and 5.3. Plant transformation experiments are performed using methods well known to persons skilled in the art.

### Example 6: Transformation of binary vectors to plant material

The binary plant transformation vectors according to Example 5, are used in plant transformation experiments according to the following procedures. The constructed binary vector is transferred by tri-parental mating to an acceptor Agrobacterium tumefaciens strain, followed by southern analysis of the ex-conjugants for verification of proper transfer of the construct to the acceptor strain, inoculation and co-cultivation of axenic explant material with the Agrobacterium tumefaciens strain of choice, selective killing of the Agrobacterium tumefaciens strain used with appropriate antibiotics, selection of transformed cells by growing on selective media containing kanamycine, transfer of tissue to shoot-inducing media, transfer of selected shoots to root inducing media, transfer of plantlets to soil, assaying for intactness of the construct by southern analyses of isolated total DNA from the transgenic plant, assaying for proper function of the inserted chimeric gene by northern analysis and/or enzyme assays and western blot analysis of proteins.

### Example 7: Expression of ATP-Related RNA Sequences in plant cells

200 mg leafmaterial are ground to a fine powder in liquid nitrogen and 800 µl RNA extraction buffer (100 mM Tris-CHl (pH 8,0), 500 mM NaCl, 2 mM EDTA, 200 mM β-Mercapto-ethanol , 0,4 % SDS) are added thereto. The homogenate is extracted with phenol, and the nucleic acids precipitated with ethanol. Resuspend the nucleic acids in 0,5 ml 10 mM Tris HCl (pH 8,0), 1 mM EDTA, add LiCl to a final concentration of 2 M, leave on ice for maximal 4 hours and collect the RNA by centrifugation. Resuspend in 400 µl 10 mMTris-HCl (pH 8,0), 1 mM EDTA and precipitate with alcohol, finally resuspend in 50 µl 10 mM Tris-HCl (pH 8.0), 1 MM EDTA. RNAs are separated on glyoxal/agarose gels and blotted to Genescreen as described by van Grinsven et al. (1986). ATP Related RNA is detected using DNA or RNA probes labeled with [³²P], [³⁵S] or by using non-radioactive labeling techniques. Based on these northern analysis, it is determined to what extent the regenerated plants express the chimeric ATP Related Sequences.

Plants transformed with chimeric constructs containing the sense "ATP Related RNA Sequences" are also subjected to western blot analysis. Proteins are extracted from leaves of transformed plants by grinding in sample buffer according to Laemmli (1970), Nature 244: 29-30.. A 50 µg portion of protein is subjected to electrophoresis in a 12,5 % SDS-polyacrylamide gel essentially as described by Laemmli (1970).Separated proteins are transferred to nitro-cellulose electrophoretically as described by Towbin H. et al. (1979), Proc. Natl. Acad. Sci USA 76: 4350-4354. Transferred proteins are reacted with antiserum raised against purified vacuolar ATPase subunit according to Towbin H. et al. supra. Based on the results of the western analysis, it is determined that transformed plants contain ATPase subunit proteins encoded by the inserted chimeric genes.

### Example 8: Increase of vacuolar pH in transgenic plants expressing ATP- Related RNA sequences

Transformed plants are grown in the greenhouse under standard quarantine conditions in order to prevent any infections by pathogens. Primary transformed plants are analysed for the presence of the inserted gene and subsequently tested for the pH of the vacuoles using methods as described hereinbefore.Analogous to Example 5.1 the original carrot 69 kd ATPase subunit is cloned into SmaI/Pst I linearized pZU-C. The chimeric CHS-ATPase-NOS terminator cassette is excised and cloned into appropriate restriction sites of pBIN19. The resulting plasmid is used in plant transformation experiments using methods well known to a person skilled in the art.
Plants containing ATP Related DNA Sequences do show increased vacuolar pH as exemplified by a spectral shift to blue compared to untransformed control plants which show their original flower colour

Selected plants containing ATP Related DNA Sequences can be self-pollinated or outcrossed to different genetic backgrounds indicating the stable inheritance of this newly introduced gene.

SEQUENCE LISTING

SEQ ID NO: 1

Sequence type: nucleotide
Sequence length: 1872 base pairs
Strandedness: single
Original Source Organism: carrot

1 ATG CCT TCT GTG TAT GGG GAT CGA TTA ACT ACG TTC GAA GAC TCC 45
46 GAG AAG GAG AGC GAG TAT GGT TAC GTC CGT AAG GTA TCC GGA CCA 90
91 GTT GTT GTC GCC GAT GGA ATG GGA GGT GCG GCT ATG TAT GAA CTT 135
136 GTT CGT GTT GGA CAT GAC AAT CTT ATT GGA GAA ATT ATT CGT TTG 180
181 GAA GGA GAT TCT GCT ACA ATC CAA GTT TAC GAA GAA ACA GCT GGG 225
226 TTG ATG GTG AAT GAT CCT GTC CTT AGG ACA CAC AAG CCC CTA TCT 270
271 GTG GAG TTG GGT CCT GGA ATT CTG GGA AAT ATC TTT GAT GGT ATT 315
316 CAG AGA CCT TTG AAG ACC ATT GCT AAG AGA TCA GGT GAT GTC TAT 360
361 ATC CCT CGC GGT GTC TCG GTA CCA GCC CTT GAC AAA GAC ACA CTA 405
406 TGG GAA TTT CAG CCT AAA AAA ATA GGT GAA GGG GAC CTT CTT ACA 450
451 GGT GGA GAT TTA TAT GCT ACG GTC TTT GAG AAT AGT TTA ATG CAA 495
496 CAT CAT GTT GCT CTG CCT CCT GAT GCC ATG GGA AAG ATA ACA TAT 540
541 GTT GCT CCA GCT GGT CAA TAT TCA CTA AAG GAT ACA GTT TTG GAG 585
586 CTT GAA TTT CAA GGC GTC AAG AAG CAA TTT ACT ATG CTT CAG ACT 630
631 TGG CCC GTA CGA ACT CCC AGG CCT GTT GCA TCA AAG CTT GCC GCT 675
676 GAC ACT CCT CTT CTC ACA GGA CAG CGT GTA CTT GAT GCC CTT TTC 720
721 CCA TCT GTG CTT GGG GGA ACT TGT GCA ATT CCT GGT GCA TTT GGT 765
766 TGT GGA AAA ACA GTC ATT AGT CAG GCT CTT TCC AAG TAT TCC AAT 810
811 TCT GAC ACT GTG GTT TAT GTT GGT TGC GGG GAA AGA GGA AAT GAA 855
856 ATG GCA GAG GTC CTT ATG GAT TTT CCT CAA TTG ACA ATG ACT CTC 900
901 CCT GAT GGC CGT GAA GAG TCT GTC ATG AAA CGT ACT ACA CTT GTG 945
946 GCC AAC ACT TCA AAC ATG CCT GTG GCT GCT CGC GAG GCT TCA ATT 990
991 TAT ACA GGA ATC ACT ATA GCA GAA TAC TTC AGA GAT ATG GGT TAC 1035
1036 AAT GTT AGT ATG ATG GCA GAT TCA ACA TCC CGA TGG GCA GAA GCT 1080
1081 TTA AGA GAA ATT TCA GGG CGA TTG GCA GAA ATG CCT GCT GAT AGT 1125
1126 GGG TAT CCT GCT TAC TTG GCA GCT CGT TTA GCG TCC TTC TAT GAG 1170
1171 CGT GCT GGT AAG GTG AAG TGC CTT GGT GGA CCA GAA CGT AAC GGT 1215
1216 AGT GTT ACT ATC GTT GGT GCT GTT TCA CCT CCA GGA GGA GAT TTT 1260

1261 TCT GAC CCT GTT ACA TCT GCC ACA CTC AGC ATC GTT CAG GTC TTT 1305
1306 TGG GGT TTA GAC AAA AAG TTG GCA CAG AGG AAA CAT TTT CCT TCT 1350
1351 GTG AAC TGG CTC ATC TCC TAT TCA AAG TAT TCA ACA GCA TTG GAG 1395
1396 TCC TTT TAT GAG AAA TTT GAT TCA GAC TTT ATC GAC ATC AGG ACA 1440
1441 AAA GCT CGT GAG GTT TTG CAG AGG GAA GAT GAT CTG AAT GAA ATT 1485
1486 GTC CAG CTG GTT GGG AAG GAT GCT CTA GCA GAA ACA GAT AAA ATT 1530
1531 ACC TTA GAG ACT GCA AAG CTT TTA AGG GAG GAT TAT CTT GCC CAG 1575
1576 AAT GCA TTT ACT CCA TAT GAC AAA TTT TGT CCT TTT TAC AAG TCT 1620
1621 GTT TGG ATG ATG CGC AAT ATT ATC CAT TTT TAC AAT CTG GCC AAT 1665
1666 CAG GCT GTG GAG CGA GGA GCT GGT ATG GAT GGC CAG AAG ATA AGT 1710
1711 TAT ACC CTT ATC AAG CAT CGT CTA GGG GAC CTT TTC TAT CGC CTG 1755
1756 GTT TCT CAG AAA TTT GAG GAT CCC GCT GAA GGT GAG GAT GTA CTT 1800
1801 GTA GGG AAG TTC AAG AAA CTT CAT GAT GAT CTT ACA TCT GGT TTC 1845
1846 CGG AAT CTT GAG GAC GAG ACA CGA TGA 1872

SEQ ID NO: 2

Sequence type: protein
Sequence length: 623 amino acids
Original Source Organism: carrot

Met pro ser val tyr gly asp arg leu thr thr phe glu asp ser 15
                5                   10                  15

glu lys glu ser glu tyr gly tyr val arg lys val ser gly pro 30
20 25 30

val val val ala asp gly met gly gly ala ala met tyr glu leu 45
35 40 45

val arg val gly his asp asn leu ile gly glu ile ile arg leu 60
50 55 60

glu gly asp ser ala thr ile gln val tyr glu glu thr ala gly 75
65 70 75

leu met val asn asp pro val leu arg thr his lys pro leu ser 90
80 85 90

val glu leu gly pro gly ile leu gly asn ile phe asp gly ile 105
95 100 105

gln arg pro leu lys thr ile ala lys arg ser gly asp val tyr 120
110 115 120

ile pro arg gly val ser val pro ala leu asp lys asp thr leu 135
125 130 135

trp glu phe gln pro lys lys ile gly glu gly asp leu leu thr 150
140 145 150

gly gly asp leu tyr ala thr val phe glu asn ser leu met gln 165
155 160 165

his his val ala leu pro pro asp ala met gly lys ile thr tyr 180
170 175 180

val ala pro ala gly gln tyr ser leu lys asp thr val leu glu 195
185 190 195

leu glu phe gln gly val lys lys gln phe thr met leu gln thr 210
200 205 210

trp pro val arg thr pro arg pro val ala ser lys leu ala ala 225
215 220 225

asp thr pro leu leu thr gly gln arg val leu asp ala leu phe 240
230 235 240

pro ser val leu gly gly thr cys ala ile pro gly ala phe gly 255
245 250 255

cys gly lys thr val ile ser gln ala leu ser lys tyr ser asn 270
260 265 270

ser asp thr val val tyr val gly cys gly glu arg gly asn glu 285
275 280 285

met ala glu val leu met asp phe pro gln leu thr met thr leu 300
290 295 300

pro asp gly arg glu glu ser val met lys arg thr thr leu val 315
305 310 315

ala asn thr ser asn met pro val ala ala arg glu ala ser ile 330
320 325 330

tyr thr gly ile thr ile ala glu tyr phe arg asp met gly tyr 345
335 340 345

asn val ser met met ala asp ser thr ser arg trp ala glu ala 360
350 355 360

leu arg glu ile ser gly arg leu ala glu met pro ala asp ser 375
365 370 375

gly tyr pro ala tyr leu ala ala arg leu ala ser phe tyr glu 390
380 385 390

arg ala gly lys val lys cys leu gly gly pro glu arg asn gly 405
395 400 405

ser val thr ile val gly ala val ser pro pro gly gly asp phe 420
410 415 420

asp pro val thr ser ala thr leu ser ile val gln val phe 435
425 430 435

leu asp lys lys leu ala gln arg lys his phe pro ser 450
440 445 450

leu ile ser tyr ser lys tyr ser thr ala leu glu 465
455 460 465

lys phe asp ser asp phe ile asp ile arg thr 480
[?]70 475 480

lys ala arg glu val leu gln arg glu asp asp leu asn glu ile 495
485 490 495

val gln leu val gly lys asp ala leu ala glu thr asp lys ile 510
500 505 510

thr leu glu thr ala lys leu leu arg glu asp tyr leu ala gln 525
515 520 525

asn ala phe thr pro tyr asp lys phe cys pro phe tyr lys ser 540
530 535 540

val trp met met arg asn ile ile his phe tyr asn leu ala asn 555
545 550 555

gln ala val glu arg gly ala gly met asp gly gln lys ile ser 570
560 565 570

tyr thr leu ile lys his arg leu gly asp leu phe tyr arg leu 585
575 580 585

val ser gln lys phe glu asp pro ala glu gly glu asp val leu 600
590 595 600

val gly lys phe lys lys leu his asp asp leu thr ser gly phe 615
605 610 615

arg asn leu glu asp glu thr arg 623
620

SEQ ID NO: 3

Sequence type:    nucleotide
Sequence length:  31 base pairs
Source:  synthetic nucleotide

CATGTTCTAG ATCATCGTGT CTCGTCCTCA AG    (SEQ ID No: 3)

SEQ ID NO: 4

Sequence type: nucleotide
Sequence length: 26 base pairs
Source: synthetic nucleotide

CTGAATCTAG AGAAGATCCC TTCTGT (SEQ ID No: 4)

SEQ ID NO: 5

Sequence type:   nucleotide
Sequence length: 453 base pairs
Source:  Chalcone synthase promoter fragment
         from Petunia hybrida

1 ATCGATGAGC TTTAAGTGCA TGTGCACTGT CAAATTATGT CATAAAATTC
51 TAATGAGATG AGTTATATAG CATTTACAAA TTCAAATACT TAAAAAGGAT
101 ACTACAATTT GGTATTACCT TGTCAAATTC CTGTTCAAAG CTGATGCTAG
151 GAGTGACAGA AATCATATGT AAGAATGATC AAGACCATTC ATTTGGTTCA
201 ACAGACTGAT GAAAAATCTA AGAGCCTTTG ATCTTGAAGA AGTAGGTAGC
251 GCTAATTCGA AAATAACCAA CCATTAATTT GTTGTGAAGG TTTGCTACGA
301 AAATAAAAAA GGATGTCACG TGCCATCAAG TTATAGCTAC ACGTGATTAC
351 TANCTACCAT TCTTCTTTTG GG[?]TCTCGTA TAAATACTTT ACAATCCCCA
401 TGCAAACCAT AACACAATAT AAAAGATACT TACACTTGTC ACGTCGCGGA
451 TCC

SEQ ID NO: 6

Sequence type:   nucleotide
Sequence length:  14 base pairs
Source:  synthetic nucleotide

CTGAGAAG ATG CCT)   (SEQ ID No: 6)

SEQ ID NO: 7

Sequence type: nucleotide
Sequence length: 14 base pairs
Source: synthetic nucleotide

CTGATATC ATG CCT (SEQ ID No: 7)

SEQ ID NO: 8

Sequence type:   nucleotide
Sequence length:  18 base pairs
Source:  synthetic nucleotide

ACACG ATGATTGAAC ATG   (SEQ ID No: 8)

SEQ ID NO: 9

Sequence type:   nucleotide
Sequence length: 18 bsae pairs
Source: synthetic nucleotide

ACACG ATGACTGCAG ATG   (SEQ ID No: 9),

## Claims

1. A DNA construct capable of influencing pigment colour in the vacuoles of flower cells which comprise a DNA sequence coding for transcription
a) into an RNA sequence which encodes a vacuolar ATPase subunit or a part thereof; or into an RNA sequence homologous with an RNA sequence which encodes a vacuolar ATPase subunit or encodes a part of a vacuolar ATPase subunit; or
b) into an RNA sequence complementary to an RNA sequence which encodes a vacuolar ATPase subunit or a part thereof; or into an RNA sequence homologous with an RNA sequence complementary to an RNA sequence which encodes a vacuolar ATPase subunit or encodes a part of a vacuolar ATPase subunit;
which DNA sequence are under expression control of a promoter capable of functioning in plants and includes a terminator capable of functioning in plants.

2. DNA constructs according to Claim 1 which when expressed in a plant genome are capable of influencing the colour of pigments in flower cell vacuoles through an alteration in vacuolar pH.

3. DNA constructs according to Claim 1 or Claim 2, wherein the pH of the vacuole is altered from being an acidic pH value towards a more basic pH value.

4. DNA constructs according to any one of the preceding claims wherein the DNA construct comprises
(i) the DNA sequence (SEQ ID No: 1) encoding the 69 kd subunit of vacuolar ATPase obtainable from carrot (SEQ ID No. 2) or a part thereof; or an equivalent DNA sequence coding for said ATPase subunit; or a DNA sequence encoding a protein homologous with the 69 kd subunit of vacuolar ATPase obtainable from carrot or a part thereof; **or**
(ii) a DNA sequence coding for transcription into an RNA sequence complementary to the RNA sequence encoding the 69 kd subunit of vacuolar ATPase obtainable from carrot, or a part thereof; or a DNA sequence coding for transcription into an RNA sequence homologous with the RNA sequence complementary to the RNA sequence encoding the 69 kd subunit of vacuolar ATPase obtainable from carrot, or a port thereof.

5. A DNA construct according to any one of Claims 1 to 4, wherein the length of the DNA sequence coding for transcription into a part of an RNA sequence encoding a vacuolar ATPase subunit (as stated under Claim 1a) is at least 30 % of the length of an ATPase subunit protein.

6. A DNA construct according to any one of Claims 1 to 5, where the DNA sequence encoding for transcription into an RNA sequence homologous with an RNA sequence which encodes a vacuolar ATPase subunit or a part of a vacuolar ATPase subunit (as stated in Claim 1a) has substantial sequence homology to a vacuolar ATPase subunit or to a part of a vacuolar ATPase subunit.

7. A DNA construct according to Claim 6, wherein the DNA sequence coding for transcription into an RNA sequence homologous with an RNA sequence encoding a part of a vacuolar ATPase subunit, codes for a protein having at least 30 % of the length of an ATPase subunit protein.

8. A DNA construct according to any one of Claims 1 to 7, coding for a protein having a length of from 80 % to 120 % of the length of an ATPase subunit protein.

9. A DNA construct according to any one of Claims 1 to 4, wherein the DNA sequence coding for transcription into an RNA sequence as stated in Claim 1b), has a length of from 50 nucleotides up to 120 % of the number of nucleotides included in the DNA sequence encoding a vacuolar ATPase subunit.

10. A transgenic plant, wherein the pigment colour has been changed by alteration of the vacuolar pH.

11. Transgenic plants according to Claim 10, wherein the altered vacuolar pH is due to inhibition of the function of vacuolar ATPase or inhibition of the synthesis thereof.

12. Transgenic plants according to Claim 10 or Claim 11, comprising in their genome a DNA sequence as stated in any one of Claim 1 to 9.

13. Transgenic plants according to any one of Claims 10 to 12, selected from the group comprising Anthirrhinum, Chrysanthemum, Cyclamen, Dahlia, Datura, Delphinium, Gerbera, Gladiolus, Gloxinia, Hippeastrum, Impatiens, Mesembryanthemum, Papaver, Pelargonium, Primula, Rosa, Saint Paulia, Salpiglossis, Helianthus (sunflower), Tagetes, Verbena and Vinca.

14. A method of obtaining plants according to any one of Claims 10 to 13, which comprises
a) inserting into the genome of a plant cell a DNA construct according to any one of Claims 1 to 9;
b) obtaining transformed cells; and
c) regenerating transformed plants from said transformed cells.
